# NOUVEAU FASCICULE DE BREVET EUROPEEN

(11) **EP 2 988 757 B2**
(45) Date de publication et mention de la décision concernant l'opposition: **12.01.2022**
(45) Mention de la délivrance du brevet: 16.01.2019
(21) Numéro de dépôt: 14720568.6
(22) Date de dépôt: 25.04.2014
(51) Int. Cl.: A61K 33/36, A61P 17/00, A61P 17/06

(54) **TRAITEMENT DES LÉSIONS CUTANÉES ASSOCIÉES À DES MALADIES AUTOIMMUNES ET INFLAMMATOIRES PAR LE COMPOSÉ DE L'ARSENIC AS2O5**
BEHANDLUNG VON HAUTLÄSIONEN MIT AUTOIMMUN- UND ENTZÜNDUNGSKRANKHEITEN MITHILFE DER ARSENVERBINDUNG AS2O5
TREATMENT OF SKIN INJURIES ASSOCIATED WITH AUTO-IMMUNE AND INFLAMMATORY DISEASES USING THE ARSENIC COMPOUND AS2O5

(30) Priorité: 26.04.2013 FR 1353870
(43) Date de publication de la demande: 02.03.2016
(60) Demande divisionnaire: 18199984.8
(73) Titulaire: Centre National de la Recherche Scientifique (C.N.R.S.), 75016 Paris (FR); Université Paris-Sud 11, 91400 Orsay (FR)
(72) Inventeur: BENBIJJA, Mohcine, 75019 Paris (FR); BOBE, Pedro, 75006 Paris (FR)
(74) Mandataire: Cabinet Becker et Associés
(86) Numéro de dépôt international: PCT/EP2014/058420
(87) Numéro de publication internationale: WO 2014/174059

(56) Documents cités:
- EP-A2- 1 736 161
- WO-A1-03/090766
- WO-A2-2008/097824
- CA-A1- 1 107 514
- US-A1- 2005 196 464
- US-A1- 2009 297 624
- DATABASE CA [Online] CHEMICAL ABSTRACTS SERVICE, COLUMBUS, OHIO, US; 21 juin 2005 (2005-06-21), DAI, ZHAOYUN: "Application of arsenic compounds in medicines for treating skin disease", XP002716868, extrait de STN Database accession no. 2005:533176 & CN 1 528 344 A (DAI ZHAOYUN [CN]) 15 septembre 2004 (2004-09-15)
- TSE WAI-PUI ET AL: "Arsenic trioxide, arsenic pentoxide, and arsenic iodide inhibit human keratinocyte proliferation through the induction of apoptosis.", THE JOURNAL OF PHARMACOLOGY AND EXPERIMENTAL THERAPEUTICS AUG 2008, vol. 326, no. 2, août 2008 (2008-08), pages 388-394, XP002716869, ISSN: 1521-0103
- TSE WAI-PUI ET AL: "Realgar-mediated growth inhibition on HaCaT human keratinocytes is associated with induction of apoptosis.", INTERNATIONAL JOURNAL OF MOLECULAR MEDICINE AUG 2009, vol. 24, no. 2, août 2009 (2009-08), pages 189-196, XP002716871, ISSN: 1107-3756
- LiuZuo et al.: "The research progress of realgar", , 29 mars 2009 (2009-03-29), XP002725755, Extrait de l'Internet: URL:http://www.yourpaper.net/article/20090 329/83374.html#.U5qt71Psx8E [extrait le 2014-06-13]
- ADACHI S ET AL: "GEL FILTRATION PROFILE OF THE ARSENIC COMPOUNDS IN WATER EXTRACTS OF SEAWEEDS", JOURNAL OF THE FOOD HYGIENIC SOCIETY OF JAPAN, vol. 21, no. 1, 1980, pages 13-17, XP002716872, ISSN: 0015-6426
- DATABASE CA [Online] CHEMICAL ABSTRACTS SERVICE, COLUMBUS, OHIO, US; 30 avril 2007 (2007-04-30), LIU, JIAYOU: "Composition of traditional Chinese medicine lotion for treating psoriasis", XP002716870, extrait de STN Database accession no. 2007:467634 & CN 1 951 440 A (LIU JIAYOU [CN]) 25 avril 2007 (2007-04-25)
- AMMANN, A. A.: "Arsenic Speciation Analysis by Ion Chromatography - A Critical Review of Principles and Applications", American Journal of Analytical Chemistry, vol. 2, 2011, pages 27-45,
- Réponse des Titulaires à la Communication selon les Règles 161 (1) et 162 CBE, datée du 8 juin 2016
- TSE, W.P. et al.: "Realgar-mediated growth inhibition on HaCaT human keratinocyte is associated with induction of apoptosis", International Journal of Molecular Medicine, vol. 24, 2009, pages 189-196, DOI: 10.3892/ijmm_00000222
- SHI, Q. et al.: "Pharmacokinetic Properties of Arsenic Species after Intravenous and Intragastrical Administration of Arsenic Trioxide Solution in Cynomolgus Macaques Using HPLC-ICP-MS", Molecules, vol. 24, no. 241, 10 January 2019 (2019-01-10), pages 1-17,
- Handbook of Inorganic Compounds
- KUROKI et al Arsenic Trioxide Inhibits Hepatitis C Virus RNA Replication through Modulation of the Glutathione Redox System and Oxidative Stress JOURNAL OF VIROLOGY. 2009
- Summery of opinion on orphan designation
- MILLER et al, "Mechanisms of Action of Arsenic Trioxide", CANCER RESEARCH 2002
- VAHTER, "Biotransformation of Trivalent and Pentavalent Inorganic arsenic in Mice and Rats" ENVIRONMENTAL RESEARCH 1981
- YODA et al." Arsenic Trioxide Augments Chk2/p53-mediated Apoptosis by inhibiting Oncogenic Wip 1 Phosphatase" THE JOURNAL OF BIOLOGICAL CHEMISTRY, 2008
- PROUST et al."Toxicologie de l arsenic et de ses composés: importance de la spéciation", L'ACTUALITÉ CHIMIQUE, 2000
- AK Scientific, Inc. - Catalog ID # 5486AF
- Aurora Fine Chemicals - Product information
- Pharmacologie médicale - vue d'ensemble notion de synergie

## Description

L'invention concerne le domaine des maladies auto-immunes et/ou inflammatoires humaines, plus précisément l'utilisation de composés de l'arsenic pour la préparation de médicament destinés au traitement et/ou à la prévention des lésions cutanées associées à des maladies de type essentiellement auto-immun ou inflammatoire ou de type auto-immun mais avec une forte composante inflammatoire responsable de leur initiation et de certaines lésions tissulaires.

Les maladies auto-immunes représentent un groupe hétérogène de maladies du système immunitaire caractérisées par la production par le sujet d'anticorps dirigés contre le soi (aussi appelés auto-anticorps), c'est-à-dire réagissant avec des antigènes du sujet lui-même. Certaines de ces maladies ont parfois aussi été classées en tant que maladies inflammatoires, dans la mesure où elles impliquent aussi des mécanismes dits dysimmunitaires.

Les maladies auto-immunes et/ou inflammatoires les plus courantes sont: le lupus systémique érythémateux, le lupus érythémateux aigu disséminé, l'uvéite, la maladie de Bechet, la sarcoïdose, le syndrome de Sjögren, la polyarthrite rhumatoïde, la polyarthrite juvénile, le syndrome de Fiessinger-Leroy-Reiter, la goutte, l'ostéoarthrose, la polymyosite, la myocardite, la cirrhose biliaire primitive, la maladie de Crohn, la colite ulcéreuse, la sclérose en plaques et autres maladies démyélinisantes, l'anémie aplasique, le purpura thrombocytopénique essentiel, toute maladie associée à une lymphoprolifération non tumorale, le lymphome à lymphocytes B, le panhypopituitarisme de Simmonds, la maladie de Basedow-Graves et l'ophtalmopathie de Graves, la thyroïdite subaiguë et la maladie de Hashimoto, la maladie d'Addison, les hépatites chroniques, le diabète sucré insulino-dépendant (type 1).

Un grand nombre de ces pathologies se caractérisent par des atteintes dermatologiques, en particulier cutanées ou cuntanéomuqueuses. C'est le cas en particulier du lupus érythémateux, de la sclérodermie, du psoriasis, de la vascularite cutanée, du purpura vasculaire, des dermatoses bulleuses auto-immunes (pemphigoïde bulleuse, pemphigoïde cicatricielle, dermatose à IgA linéaire, dermatite herpétiforme, épidermolyse bulleuse acquise, pemphigus et ses variants), des dermatites (atopique, séborrhéique, de stase), de la dermatomyosite, de l'érythème noueux, du *pyoderma gangrenosum,* de l'eczéma (atopique, de contact, dishydrosique), du lichen plan, du lichen scléro-atrophique, de l'alopécie areata.

Le lupus érythémateux en particulier est une maladie systémique auto-immune chronique, de la famille des connectivités, qui se manifeste différemment selon les individus. Cette pathologie a fait l'objet de nombreuses recherches, et est souvent utilisée comme modèle d'étude pour l'ensemble des maladies auto-immunes et/ou inflammatoires humaines présentant des lésions cutanées mentionnées ci-dessus.

Dans le lupus érythémateux on distingue le lupus érythémateux cutané (LEC) et le lupus érythémateux disséminé (LED) ou systémique (LES). Le LEC est une affection particulièrement polymorphe classiquement divisée en trois groupes : LEC chronique qui regroupe les lupus discoïde, *tumidus, pernio* et profond (ou panniculite), en LEC subaigu (LECS) et LEC aigu (LECA).

De nombreuses manifestations dermatologiques sont également observées au cours du LES. Elles peuvent être schématiquement classées en trois groupes: les lésions lupiques (histologie évocatrice de lupus), les lésions vasculaires (vascularite cutanée) et les autres manifestations. L'examen anatomopatholoqique d'une lésion cutanée lupique révèle des lésions épidermiques et dermiques : hyperkératose, atrophie épidermique, dégénérescence des kératinocytes basaux, épaississement de la membrane basale, oedème et infiltrat dermique composé essentiellement de lymphocytes.

La prise en charge du LES nécessite un traitement de fond, généralement à base de hydroxychloroquine, de chloroquine ou de corticoïdes par voie systémique. (Liste des actes et prestations ALD 21 - « Lupus érythémateux systémique », HAS Janvier 2012). Dans le cas des lupus érythémateux discoïde et cutané subaigu, le traitement en première intention est basé sur l'application locale d'immunosuppresseurs habituellement utilisés en dermatologie, tel que les corticoïdes ou le Tacrolimus. En cas d'échec des traitements locaux, des traitements systémiques à base d'hydroxychloroquine et/ou de chloroquine, ou encore de Thalidomide (voire de Méthotrexate dans les formes sévères de la maladie, en particulier avec l'objectif de limiter l'emploi de la cortisone), sont administrés.

Lorsqu'ils existent, les traitements des maladies auto-immunes et/ou inflammatoires humaines présentant des lésions cutanées autres que le lupus citées plus haut, en particulier la sclérodermie, le psoriasis, la vascularite cutanée, le purpura vasculaire, les dermatoses bulleuses auto-immunes (en particulier la pemphigoïde huileuse, la pemphigoïde cicatricielle, la dermatose à IgA linéaire, la dermatite herpétiforme, l'épidermolyse bulleuse acquise, le pemphigus et ses variants), les dermatites, (en particulier la dermatite atopique, la dermatite séborrhéique, la dermatite de stase), la dermatomyosite, l'érythème noueux, le *pyoderma gangrenosum,* l'eczéma (en particulier l'eczéma atopique, l'eczéma de contact, l'eczéma dishydrosique), le lichen plan, le lichen scléro-atrophique, et l'alopécie areata.

Ces traitements présentent cependant des inconvénients notoires. De façon générale, les immunosuppresseurs, en diminuant les réponses immunitaires de façon non-spécifique, exposent l'organisme à des complications infectieuses virales ou bactériennes et, à plus long terme, au développement d'affections malignes.

Par ailleurs, la toxicité oculaire de la chloroquine et de l'hydroxychloroquine, conduisant principalement à l'atteinte de la cornée et la rétinopathie, est décrite depuis longtemps. La rétinopathie est parfois irréversible, et peut entraîner une réduction importante de la vision.

Les effets secondaires de l'administration des corticoïdes tant en application systémique que locale sont eux aussi bien connus. En particulier, l'administration topique de corticoïde est associée à la fois à une augmentation des risques d'infection et à une atrophie de l'épiderme, ainsi qu'aux phénomènes de tachyphylaxie et d'effet rebond. Le phénomène de tachyphylaxie se traduit en pratique par l'apparition d'une tolérance, et à terme d'une résistance de la dermatose au traitement après applications prolongées et ininterrompues. L'effet rebond correspond à la recrudescence de la dermatose à l'arrêt brutal des dermocorticoïdes, après traitement prolongé.

Les effets indésirables du Tacrolimus observés consistent surtout en une sensation de brûlure, des démangeaisons ou un érythème au site d'application, tandis que les effets indésirables à long terme sont encore peu connus. Quant au Méthotrexate, l'importance de ses effets indésirables, à savoir une diminution des globules blancs, des problèmes digestifs (nausées, douleurs, vomissements, aphtes), des atteintes des poumons et du foie, des infections et une perte de cheveux, explique que son utilisation reste exceptionnelle.

L'utilisation de composés de l'arsenic par voie systémique pour traiter des pathologies auto-immunes a été décrite (EP 1 499 330 ou US 2009/0297624). Ces traitements systémiques permettent une résorption des lésions cutanées du lupus. Cependant, cette utilisation systémique a pour inconvénient d'être générale et non spécifique. L'application locale de composés de l'arsenic paraît contre-indiquée dans le cas de lésions cutanées, tant il est connu que ces composés, et en particulier As₂O₃, sont des produits irritants, causant en cas de contact avec la peau un rougissement, voire même des brûlures. Le contact prolongé et répété à des solutions diluées d'As₂O₃ est connu pour causer l'irritation de la peau et la dermatite.

Il existe donc un besoin de composés pour des traitements alternatifs des maladies auto-immunes et/ou inflammatoire, en particulier présentant des lésions cutanées ou cutanéomuqueuses.

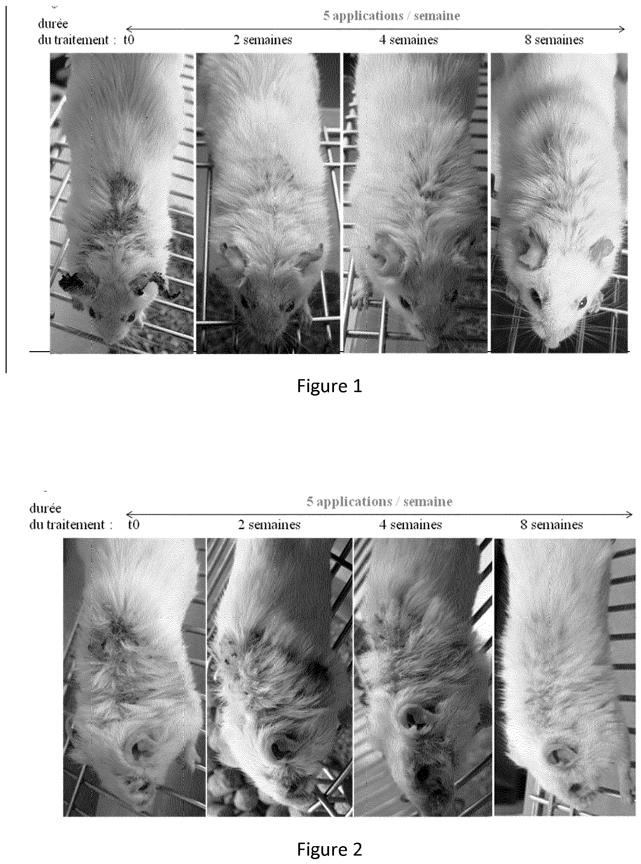

Figure 1. Traitement topique des atteintes cutanées de la souris MRL/lpr par le composé d'arsenic As₂O₃. Evolution des lésions inflammatoires du tissu cutané, de la vascularite cutanée nécrosante, et de l'alopécie chez une souris MRL/lpr femelle après 2, 4 et 8 semaines de traitement topique par 5 applications hebdomadaires du composé d'arsenic AS₂O₃ à la concentration de 0,5 mg/ml.
Figure 2. Traitement topique des atteintes cutanées de la souris MRL/lpr par le composé d'arsenic As₂O₃. Evolution des lésions inflammatoires du tissu cutané chez une souris MRL/lpr mâle après 2, 4 et 8 semaines de traitement topique par 5 applications hebdomadaires du composé d'arsenic AS₂O₃ à la concentration de 0,5 mg/ml.

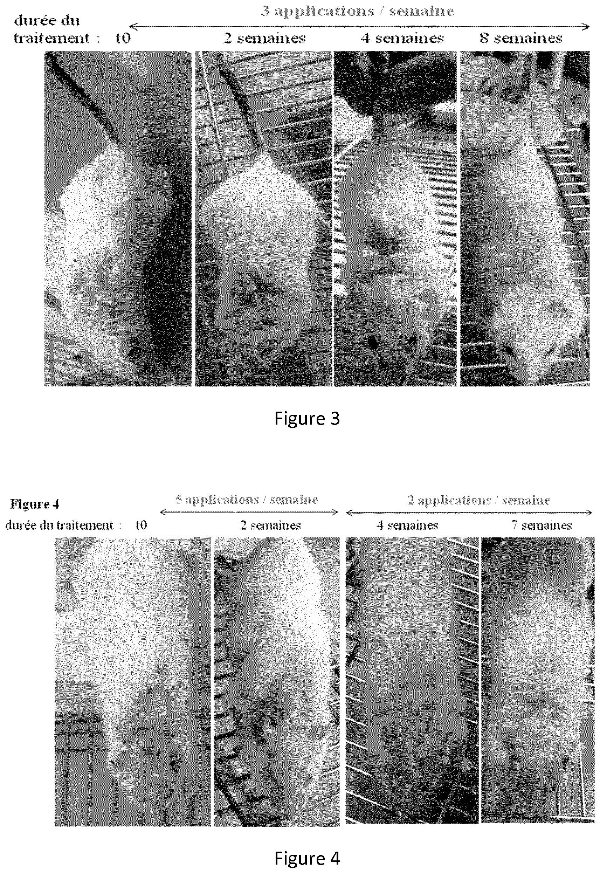

Figure 3. Traitement topique des atteintes cutanées de la souris MRL/lpr par un nombre plus réduit d'applications du composé d'arsenic As₂O₃. Evolution des lésions inflammatoires du tissu cutané et de la vascularite cutanée nécrosante chez une souris MRL/lpr femelle après 2, 4 et 8 semaines de traitement topique par le composé d'arsenic As₂O₃, à la concentration de 0,5 mg/ml, à raison de 3 applications hebdomadaires.
Figure 4. Décroissance progressive du nombre hebdomadaire d'applications topiques du composé d'arsenic As₂O₃. Evolution des lésions inflammatoires du tissu cutané chez une souris MRL/lpr traitée par le composé d'arsenic As₂O₃, à la concentration de 0,5 mg/ml, d'abord à raison de 5 applications par semaine pendant 2 semaines puis de 2 applications par semaine pendant 5 semaines supplémentaires.

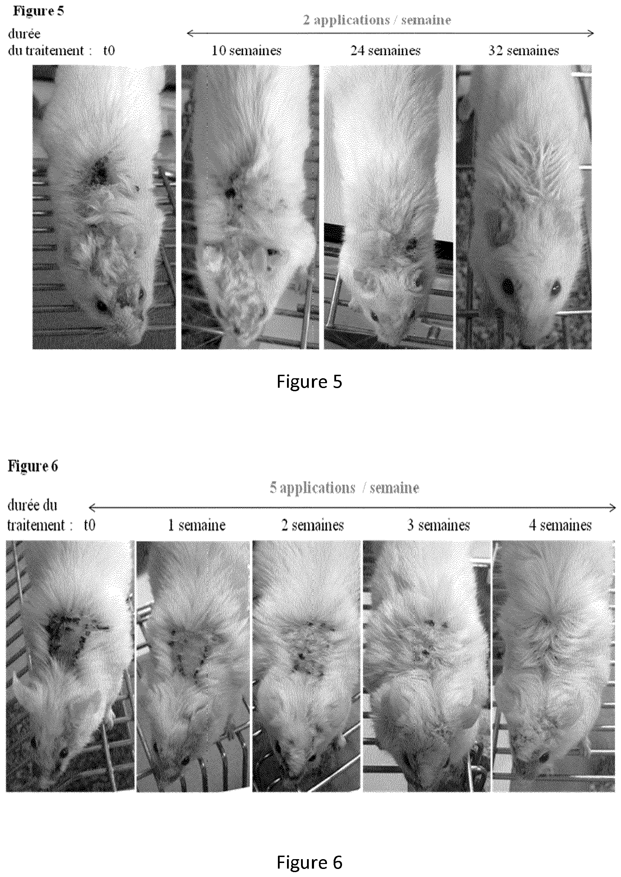

Figure 5. Le traitement topique par le composé d'arsenic AS₂O₃ entraine une augmentation significative de la durée de vie. Evolution des lésions inflammatoires du tissu cutané chez une souris MRL/lpr femelle traitée de façon discontinue pendant 32 semaines par le composé d'arsenic As₂O₃. Après 4 semaines de traitement, à raison de 5 applications par semaine, le nombre hebdomadaire d'applications du composé d'arsenic AS₂O₃ a été ramené d'abord à 3 pendant 6 semaines puis à 2 durant 22 semaines supplémentaires.
Figure 6. Traitement topique des atteintes cutanées de la souris MRL/lpr par le composé d'arsenic As₂O₅. Evolution des lésions inflammatoires du tissu cutané chez une souris MRL/lpr mâle traitée par 5 applications hebdomadaires du composé d'arsenic As₂O₅ à la concentration de 0,5 mg/ml.

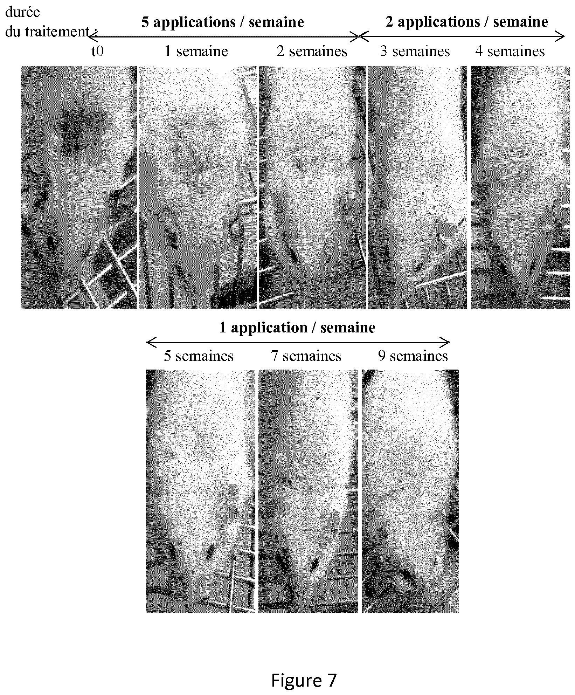

Figure 7. Décroissance progressive du nombre hebdomadaire d'applications topiques du composé d'arsenic As₂O₅. Evolution des lésions inflammatoires du tissu cutané chez une souris MRL/lpr femelle traitée par le composé d'arsenic As₂O₅ à raison d'abord de 5 applications par semaine pendant 2 semaines puis de 2 applications hebdomadaires durant 2 nouvelles semaines et enfin d'une seule application hebdomadaire pendant 5 semaines supplémentaires.

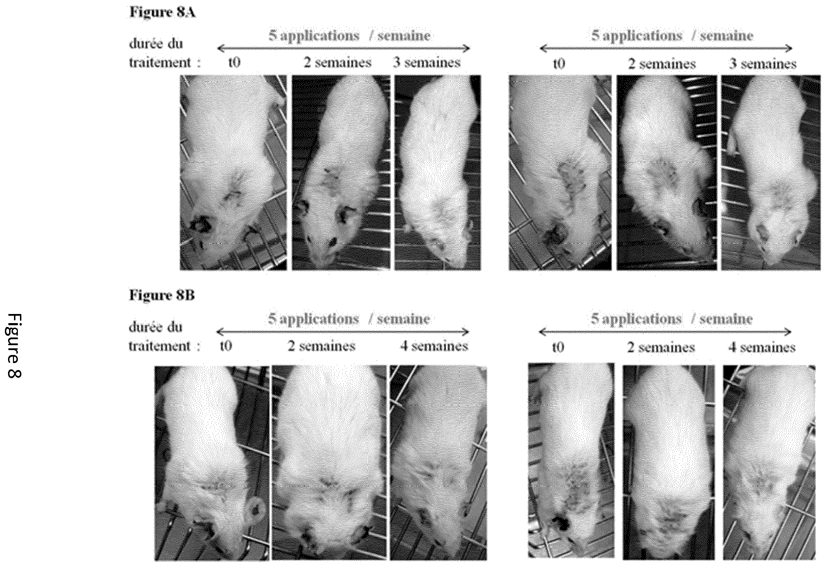

Figure 8. Traitement par la voie systémique des atteintes cutanées de la souris MRL/lpr par le composé d'arsenic As₂O₅. Evolution des lésions inflammatoires du tissu cutané chez deux souris MRL/lpr mâles (figure 8A) et deux souris femelles (figure 8B) traitées chaque semaine par 5 injections intrapéritonéales de 0,1 ml par 10 g de souris d'une solution d'arsenic As₂O₅ à la concentration de 0,5 mg/ml.

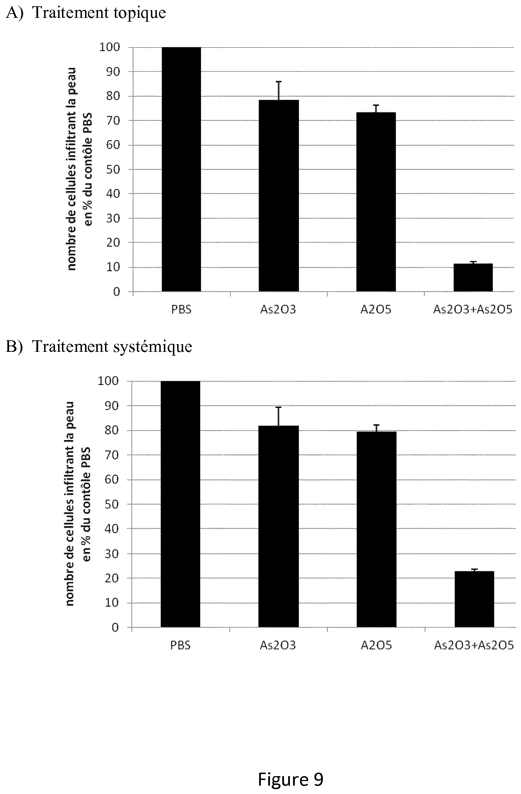

Figure 9 Quantification de l'infiltrat inflammatoire cutané chez des souris MRL/lpr traitées par seulement le composé d'arsenic As₂O₅ ou As₂O₃ ou par l'association des deux composés As₂O₃ + As₂O₅. Le nombre de cellules inflammatoires infiltrant la peau des souris MRL/lpr (3 femelles par groupe) a été quantifié après 2 mois de traitement par la voie locale (figure 9A) ou la voie systémique (figure 9B) raison de 5 applications hebdomadaires d'une solution à 0,12 mg/ml d'As₂O₃ ou d'As₂O₅ (doses suboptimales) contre 0,5 mg/mi dans les Figures 1 à 8.

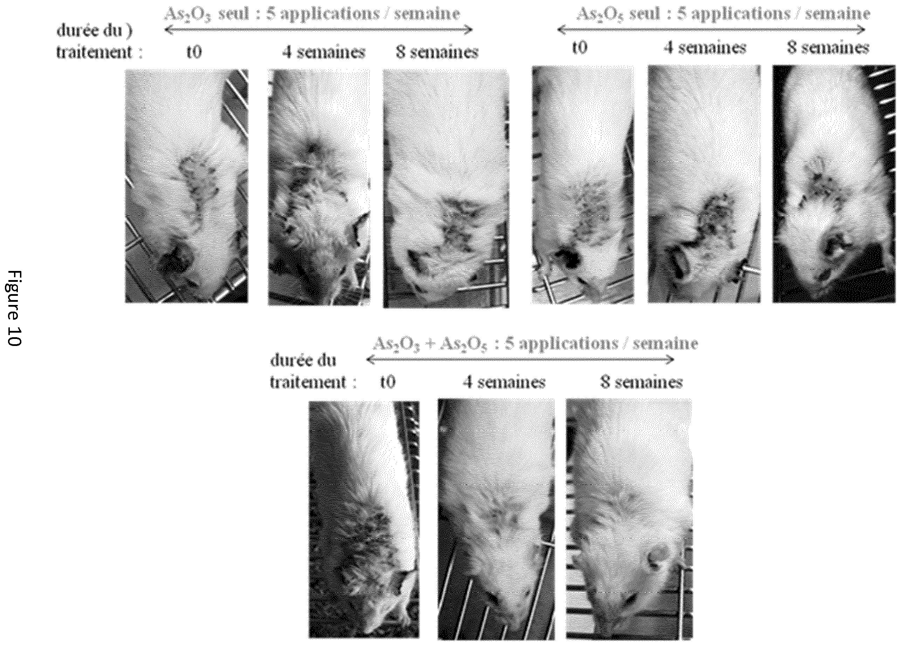

Figure 10 Traitement local des atteintes cutanées inflammatoires de la souris MRL/Ipr par l'association des deux composés As₂O₃ + As₂O₅. Evolution des lésions cutanés inflammatoires des souris MRL/Ipr femelles après 0(t0), 4 et 8 semaines d'application d'une compresse inhibée d'une solution contenant une dose suboptimale (0,12 mg/ml) d'As₂O3₅ ou d'As₂O₅ ou de l'association des deux composés As₂O₃ + As₂O₅ et cela à raison de 5 applications par semaine.

L'invention propose des moyens permettant de remédier aux inconvénients de l'art antérieur, en particulier aux effets secondaires sur le système immunitaire ainsi qu'à l'effet rebond observé dans les traitements de l'art antérieur. En effet, les Inventeurs ont découvert, de façon inattendue, que le composé de l'arsenic As₂O₅ s'avère être un excellent principe actif efficace et ne présentant pas d'effets secondaires dans le traitement et/ou la prévention des lésions cutanées associées à des maladies auto-immunes et/ou inflammatoires.

Cette utilisation médicale est d'autant plus surprenante que la toxicité du composé de l'arsenic As₂O₅ pour l'homme est notoire, la dose orale létale pour l'homme étant évaluée à 5-50 mg/kg, soit entre 7 gouttes et 1 cuillerée à café pour un sujet d'environ 75 kilos. Une exposition chronique peut provoquer des lésions nerveuses dans les extrémités, modifier la composition cellulaire du sang et provoquer des changements structurels dans les composants sanguins. Le composé de l'arsenic As₂O₅ a été décrit comme irritant pour les yeux, le nez et la gorge, et il paraît *a priori* évident qu'il serait contre-indiqué dans le cas de problèmes dermatologiques.

L'invention vise une composition comprenant une combinaison de composés de l'arsenic, caractérisée en ce qu'elle comporte entre 0,01% et 0,15% du composé d'arsenic As₂O₅ par rapport au poids total de la composition, et entre 0,05% et 5% du composé d'arsenic As₂O₃ par rapport au poids total de la composition.
L'invention vise également une composition comprenant le composé de l'arsenic As₂O₅ et le composé de l'arsenic As₂O₃, pour utilisation dans le traitement et/ou la prévention des lésions cutanées associées à des maladies auto-immunes chez un sujet humain.
Selon un autre objet, l'invention vise des produits caractérisés en ce qu'ils contiennent un composé de l'arsenic As₂O₅ et le composé de l'arsenic supplémentaire As₂O₃, comme produit de combinaison pour une utilisation simultanée, séparée ou étalée dans le temps dans le traitement et/ ou la prévention des lésions cutanées associées à des maladies auto-immunes et/ou inflammatoires chez un sujet humain.

Selon un premier aspect, il est décrit un composé de l'arsenic As₂O₅ pour utilisation dans le traitement et/ou la prévention des lésions cutanées associées des maladies auto-immunes et/ou inflammatoires chez un sujet humain.

En d'autres termes, il est décrit l'utilisation du composé de l'arsenic As₂O₅ pour la préparation d'un médicament pour le traitement et/ou la prévention des lésions cutanées associées à des maladies auto-immunes et/ou inflammatoires chez un sujet humain, ainsi qu'une méthode de prévention et/ou de traitement des lésions cutanées associées à des maladies auto-immunes et/ou inflammatoires comprenant l'administration du composé de l'arsenic As₂O₅ à un sujet humain.

En particulier, on ciblera les lésions cutanées associées aux maladies auto-immunes et/ou inflammatoires choisies parmi en particulier le lupus érythémateux, la sclérodermie, le psoriasis, la vascularite cutanée, le purpura vasculaire, les dermatoses bulleuses auto-immunes (en particulier la pemphigoïde bulleuse, la pemphigoïde cicatricielle, la dermatose à IgA linéaire, la dermatite herpétiforme, l'épidermolyse bulleuse acquise, le pemphigus et ses variants), les dermatites, (en particulier la dermatite atopique, la dermatite séborrhéique, la dermatite de stase), la dermatomyosite, l'érythème noueux, le *pyoderma gangrenosum,* l'eczéma (en particulier l'eczéma atopique, l'eczéma de contact, l'eczéma dishydrosique), le lichen plan, le lichen scléro-atrophique, et l'alopécie areata. Préférentiellement les lésions cutanées associées à des maladies auto-immunes ou inflammatoires sont des lésions cutanées associées au lupus.

Au sens de la présente invention, le terme « lupus » équivaut au terme « lupus érythémateux » et comprend le lupus érythémateux cutané (LEC) et le lupus érythémateux disséminé (LED) ou systémique (LES). Le LEC est une affection particulièrement polymorphe classiquement divisée en trois groupes : LEC chronique qui regroupe les lupus discoïde, *tumidus, pernio* et profond (ou panniculite), LEC subaigu (LECS) et LEC aigu (LECA).

Le composé de l'arsenic est particulièrement intéressant en ce qu'il n'induit pas d'effet indésirable, ni d'effet rebond. Il est donc particulièrement indiqué chez des populations particulières de sujets comme alternative à la corticothérapie, tels que les sujets non répondeurs ou présentant une tachyphylaxie à la cortisone.

Le composé de l'arsenic As205, ou pentoxyde de diarsenic, est largement utilisé comme herbicide et xyloprotecteur, ainsi que dans la fabrication de verre coloré. Il est donc aisé pour l'homme du métier de se procurer ce composé, qui peut aussi être synthétisé à partir du composé As203, ou trioxyde de diarsenic, selon des méthodes bien connues.

Selon un deuxième aspect, il est décrit une composition comprenant un composé de l'arsenic As₂0₅, préférentiellement pour le traitement et/ou la prévention des lésions cutanées associées à des maladies auto-immunes chez un sujet humain.

Le composé, ainsi que la composition, peut être administré selon une variété de modes d'administration, y compris les administrations systémiques et topiques. Le composé, ainsi que la composition, de la présente invention peut être administré par exemple par voie générale (administration systémique) qui englobe la voie orale, la voie parentérale, la voie nasale, la voie rectale ou encore la voie pulmonaire soit par voie locale (administration topique). L'homme du métier comprendra que des formulations particulières peuvent être envisagées selon le mode d'administration choisi.

La composition est préférentiellement sous une forme appropriée pour une administration topique, en particulier dermatologique. Elle peut avantageusement se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules (nanosphères, nanocapsules, vésicules lipidiques), d'un dispositif trans-dermique ou sous toute autre forme pour application topique.

Cette composition peut être plus ou moins fluide et avoir l'aspect d'une crème, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut aussi être appliquée au moyen d'un patch.

Dans le cadre d'une application topique, cette composition peut comprendre en outre un transporteur ou excipient pharmaceutiquement acceptable, en particulier un transporteur ou excipient pharmaceutiquement acceptable pour l'administration topique, notamment dermatologiquement acceptable, c'est à dire un support compatible avec la peau. De préférence, on utilise un excipient adapté pour une administration par voie topique, avantageusement adapté pour une administration dermatologique.

Les excipients dermatologiquement acceptables peuvent être tout excipient parmi ceux connus de l'homme de l'art en vue d'obtenir une composition pour l'application topique sous forme de crème, d'une lotion, d'un gel, d'une pommade, d'une émulsion, d'une microémulsion, d'un spray, etc.
La composition peut en particulier contenir des additifs et aides la formulation, tels que de l'eau, des corps gras, des tensio-actifs (émulsionnants et détergents), des épaississants, des gélifiants, des fixateurs d'eau, des agents d'étalement, des stabilisants, des colorants, des parfums et des conservateurs (antioxydant, bactéricide ou bactériostatique). Les émulsionnants appropriés comprennent par exemple l'acide stéarique, la trolamine ou triéthanolamine, le PEG-40-stéarate.

Selon un autre mode de réalisation de l'invention, la composition est sous une forme appropriée pour une administration systémique, en particulier parentérale. Elle peut avantageusement se présenter sous toutes les formes galéniques normalement utilisées pour une application parentérale, notamment sous forme d'une solution liquide, en particulier aqueuse. Préférentiellement, la composition sous une forme appropriée pour une administration parentérale est stérile. Préférentiellement, la composition sous une forme appropriée pour une administration parentérale est apyrogène. Par apyrogène, on entend au sens de l'invention que la composition selon l'invention ne donne pas de fièvre au sujet dans les 48 heures suivant son administration. Préférentiellement, la composition sous une forme appropriée pour une administration parentérale est limpide. Par limpide, on entend au sens de la présente invention qu'elle ne comprend pas de particules en suspension après avoir été inspectée visuellement. Le cas échéant, ou pour prévenir une éventuelle contamination particulaire, la composition sera filtrée à travers un filtre stérile de 0,22 IttM. Ainsi, selon un mode de réalisation particulier, la composition sous une forme appropriée pour une administration parentérale est filtrée travers un filtre stérile dont les pores ont une taille ne laissant passer que les molécules de 0,22 [tM ou moins. Préférentiellement, la composition sous une forme appropriée pour une administration parentérale a un pH compris entre 7,3 et 7,5.

L'administration parentérale peut être réalisée par injection de la composition de l'invention selon toute voie, par exemple par voie sous-cutanée, intradermique, intramusculaire, intraveineuse, intrapéritonéale, intravasculaire, injection intra-artérielle. Les injections peuvent être faites avec des seringues et des aiguilles conventionnelles, ou d'autres dispositifs appropriés disponibles dans l'art.

Les modes d'administration, les posologies et les formes galéniques optimales des composés et compositions de l'invention peuvent être déterminés selon les critères généralement pris en compte dans l'établissement d'un traitement pharmaceutique, en particulier topique et/ou dermatologique, adapté à un patient, comme par exemple l'âge ou le poids corporel du patient, la gravité de son état général, la tolérance au traitement, les effets secondaires constatés.

La dose thérapeutique utilisée dans le traitement des lésions cutanéo-muqueuses varie selon la gravité ou l'étendue des lésions et selon les conditions de traitement. Ainsi, la dose, et le cas échéant la fréquence, peuvent être adaptées en fonction de l'âge, du poids corporel, de l'étendue et de l'ancienneté des lésions. De plus, la dose et la fréquence de l'administration peuvent être adaptées de façon à obtenir une quantité efficace donnée. Selon un mode préféré de l'invention, la quantité efficace quotidienne correspond à 0,01 à 5 mg de composé d'arsenic As₂O₅ par 1kg de poids corporel du sujet, de préférence, 0,025 à 0,75 mg de composé d'arsenic As₂O₅ par 1kg de poids corporel du sujet.

La composition sous une forme appropriée pour une administration topique est typiquement administrée quotidiennement, pendant une période de 5 à 60 jours à une concentration en composé d'arsenic As₂O₅ variant de 0,5 mg (ou 0,05%) à 50 mg (ou 5%) par ml ou g de composition. Selon un mode préféré de l'invention, la quantité efficace quotidienne dans le cas d'une administration topique correspond à 0,05 à 5 mg de composé d'arsenic As₂O₅ par 1kg de poids corporel du sujet, de préférence, 0,1 à 0,5 mg de composé d'arsenic As₂O₅ par 1kg de poids corporel du sujet. La quantité totale de composition appliquée quotidiennement dépend de la surface cutanée à traiter. Préférentiellement, la quantité totale quotidienne est inférieure ou égale à 1 mg de composé d'arsenic As₂O₅ par kg de poids corporel du sujet. Il est possible d'effectuer des traitements successifs, avantageusement espacés de 1 à 4 semaines pendant lesquelles le sujet n'est pas traité.

Le traitement est préférentiellement réalisé sur 5 jours. La posologie pourra cependant être réduite en cas de lésions tissulaires de moindre ampleur. De plus, après un traitement d'attaque à forte concentration du composé d'arsenic As₂O₅ seul ou en association avec As₂O₃, un espacement des doses pourra être réalisé avec même des interruptions du traitement pouvant varier de 1 à 8 semaines selon les cas.

La composition sous une forme appropriée pour une administration parentérale est typiquement administrée quotidiennement, pendant une période de 5 à 30 jours. Il est possible d'effectuer des traitements successifs, avantageusement espacés de 1 à 8 semaines pendant lesquelles le sujet n'est pas traité. La quantité efficace quotidienne dans le cas d'une administration parentérale correspond à 0,01 à 1 mg de composé d'arsenic As₂O₅ par 1kg de poids corporel du sujet, de préférence, 0,025 à 0,75 mg de compose d'arsenic As₂O₅ par 1kg de poids corporel du sujet. Préférentiellement, la composition de l'invention comporte entre 0,01% et 0,15% du composé d'arsenic As₂O₅ par rapport au poids total de la composition.

En outre, le composé de l'arsenic As₂O₅ peut être administre le cas échéant avec d'autres principes actifs contribuant au traitement des pathologies ciblées, dans le but d'améliorer la réponse thérapeutique de sujet.

Ainsi, selon un mode de réalisation particulier, la composition décrite comporte en outre au moins un composé choisi parmi les corticoïdes, le Tacrolimus, l'hydroxychloroquine, la chloroquine, le Thalidomide, le Méthatroxate, les composés ayant une action anti-TNF, les composés ayant une action anti-CD20, les composés ayant une action anti-BAFF, les composés ayant une action anti-IL1, les composés ayant une action anti-IL-6.

Un certain nombre de composés de l'arsenic ont démontré leur intérêt dans le traitement de maladies auto-immunes, notamment le lupus (EP 1499 330) Ainsi, le composé de l'arsenic As₂O₅ peut être administré en combinaison avec d'autres composés de l'arsenic contribuant au traitement des pathologies auto-immunes et /ou inflammatoires.

Selon un autre mode de réalisation particulier, la composition décrite comporte en outre au moins un composé choisi parmi les composés de l'arsenic As₂O₃, As₄O₆, As₂S₂, As₂S₃, As₂S₅ et As₄S₄. Préférentiellement, la composition de l'invention comprenant un composé de l'arsenic As₂0₅ comporte en outre au moins le composé de l'arsenic As₂O₃.

Autrement dit, selon un autre mode de réalisation, la composition décrite comprend une combinaison de composés de l'arsenic, laquelle combinaison comprend le composé de l'arsenic As₂O₅, et en outre au moins un composé de l'arsenic supplémentaire choisi parmi la liste consistant en As₂O₃, As₂O₅, As₄O₆, As₂S₂, As₂S₃, As₂S₅ et As₄S₄. Avantageusement, ladite combinaison comprend le composé de l'arsenic As₂0₅ et le composé de l'arsenic supplémentaire AS₂O₃. Encore plus avantageusement, ladite combinaison consiste en le composé de l'arsenic As₂O₅ et le composé de l'arsenic supplémentaire As₂O₃.

La solubilité dans l'eau des différents composés inorganiques de l'arsenic varie considérablement. Par exemple, le composé d'arsenic As₂O₃ est peu soluble dans l'eau (37 g/L à 20°C) ou l'alcool, et sa formulation s'avère donc difficile. Les Inventeurs ont découvert que la solubilité du composé d'arsenic As₂O₃ peut être modifiée en variant les conditions d'oxydoréduction, de pH et de température lors de la préparation de la composition. Par exemple, pour contourner le problème de la faible solubilité du composé As₂O₃ dans l'eau, celui-ci est d'abord dissout à une forte concentration (60 à 70 mg/ml) dans une solution alcaline diluée (0,5 M ou 1 M), et en chauffant légèrement, jusqu'à obtenir une solution limpide et incolore.

Ainsi, selon un mode de réalisation particulier de l'invention, la composition décrite comporte en outre au moins le composé de l'arsenic As₂O₃ et est caractérisée en ce que le composé As₂O₃ dans ladite composition est susceptible d'être obtenu par un procédé comprenant une étape de dissolution dans une solution alcaline. Au sens de la présente invention, les termes « solution alcaline » se réfèrent à toute solution basique, c'est-à-dire toute solution dont le pH est supérieur à 7.

La solution de As₂O₃ limpide peut en outre être rendue exempte de particules et stérile par filtration, selon toute technique bien connue de l'homme du métier. La solution limpide d'As₂O₃ ainsi obtenue peut de plus être diluée dans un tampon phosphate ou dans du sérum physiologique stérile à la concentration variant de 0,5 à 1,5 mg/ml en vue de son utilisation *in vivo.*

Préférentiellement, la composition de l'invention comporte entre 0,01% et 0,15% du composé d'arsenic As₂O₅ par rapport au poids total de la composition, et entre 0,05% et 5% du composé d'arsenic As₂O₃ par rapport au poids total de la composition.

Il apparaîtra évident à l'homme du métier que l'administration combinée de plusieurs principes actifs peut être réalisée, selon les pathologies ciblées et leur gravité, via l'administration conjointe de ces composés, ou alternativement via une administration alternée de chacun d'entre eux séparément par exemple.

Selon un mode de réalisation particulier, il est décrit des produits contenant un composé de l'arsenic As₂O₅ et au moins un composé choisi parmi les corticoïdes, le Tacrolimus, l'hydroxychloroquine, la chloroquine, le Thalidomide, le Méthatroxate, les composés ayant une actionanti-TNF, les composés ayant une action anti-CD20, les composés ayant une action anti-BAFF, les composés ayant une action anti-IL1, les composés ayant une action anti-IL-6 comme produit de combinaison pour une utilisation simultanée, séparée ou étalée dans le temps dans le traitement et/ou la prévention des lésions cutanées associées à des maladies auto-immunes chez un sujet humain.

Selon un mode de réalisation particulier, il est décrit des produits contenant un composé de l'arsenic As₂O₅ et au moins un composé de l'arsenic choisi parmi les composés de l'arsenic As₂O₃, As₄O₆, As₂S₂, As₂S₃, As₂S₅ et As₄S₄, comme produit de combinaison pour une utilisation simultanée, séparée ou étalée dans le temps dans le traitement et/ ou la prévention des lésions cutanées associées à des maladies auto-immunes chez un sujet humain. Autrement dit, il est décrit des produits contenant un composé de l'arsenic As₂O₅ et un composé de l'arsenic supplémentaire choisi parmi la liste consistant en As₂O₃, As₄O₅, As₄O₆, As₂S₂, As₂S₃, As₂S₅ et As₄S₄ comme produit de combinaison pour une utilisation simultanée, séparée ou étalée dans le temps dans le traitement et/ ou la prévention des lésions cutanées associées à des maladies auto-immunes chez un sujet humain. Préférentiellement, lesdits produits de l'invention contiennent un composé de l'arsenic As₂0₅ et le composé de l'arsenic supplémentaire As₂O₃.

En d'autres termes, il est décrit des produits contenant un composé de l'arsenic As₂O₅ et au moins un composé de l'arsenic choisi parmi les composés de l'arsenic As₂O₃, As₄O₆, As₂S₂, As₂S₃, As₂S₅ et As₄S₄, comme produit de combinaison pour une utilisation simultanée, séparée ou étalée dans le temps pour la préparation d'un médicament pour le traitement et/ou la prévention des lésions cutanées associées à des maladies auto-immunes et/ou inflammatoires chez un sujet humain.

De plus, il est décrit une méthode de prévention et/ou de traitement des lésions cutanées associées à des maladies auto-immunes et/ou inflammatoires comprenant l'administration simultanée, séparée ou étalée dans le temps du composé de l'arsenic As₂O₅ et d'au moins d'un composé de l'arsenic choisi parmi les composés de l'arsenic As₂O₃, As₄O₆, As₂S₂, As₂S₃, As₂S₅ et As₄S₄ à un sujet humain. Selon un mode de réalisation particulier, il est décrit une méthode de prévention et/ou de traitement des lésions cutanées associées à des maladies auto-immunes et/ou inflammatoires comprenant l'administration du composé As₂O₅ alternée avec l'administration d'au moins d'un composé de l'arsenic choisi parmi les composés de l'arsenic As₂O₃, As₄O₆, As₂S₂, As₂S₃, As₂S₅ et As₄S₄.

En d'autres termes, il est décrit une méthode pour le traitement et/ou la prévention des lésions cutanées associées à des maladies auto-immunes et/ou inflammatoires chez un sujet humain, comprenant une étape d'administration audit sujet d'une composition ou des produits selon l'invention. Préférentiellement, l'application d'As₂O₅ est alternée avec l'administration d'au moins un composé de l'arsenic supplémentaire choisi parmi la liste consistant en As₂O₃, As₂O₅, As₄O₆, As₂S₂, As₂S₃, As₂S₅ et As₄S₄. Avantageusement, l'application d'As₂O₅ est alternée avec l'administration du composé de l'arsenic AS₂O₃.

L'administration combinée du composé de l'arsenic As₂0₅ et du composé de l'arsenic As₂O₃ en particulier permet de contourner une éventuelle résistance du sujet l'un ou l'autre de ces composés de sels d'arsenic, dans la mesure où leurs mécanismes d'action sont différents.

Selon un mode de réalisation préféré, l'invention a pour objet des produits contenant un composé de l'arsenic As₂O₅ et un composé de l'arsenic As₂O₃ comme produit de combinaison pour une utilisation simultanée, séparée ou étalée dans le temps dans le traitement et/ ou la prévention des lésions cutanées associées à des maladies auto-immunes chez un sujet humain.

Préférentiellement, les produits contenant un composé de l'arsenic As₂0₅ et un composé de l'arsenic As₂O₃ comme produit de combinaison sont administrés à une dose quotidienne pour le composé d'arsenic As₂O₅ comprise entre 0,01 et 0,2 mg pour 1kg de poids corporel du sujet et à une dose quotidienne du composé d'arsenic d'As₂O₃ comprise entre 0,01 et 0,2 mg pour 1kg de poids corporel du sujet.

Il est également décrit des nécessaires ou kits comprenant au moins un contenant renfermant le composé d'arsenic As₂O₅ sous une forme pharmaceutiquement acceptable pour son administration topique et/ou dermatologique, et avantageusement une notice d'utilisation. Le kit peut en outre comprendre un contenant renfermant le composé de l'arsenic As₂O₃. Le kit peut en outre comprendre des moyens d'application appropriés tels qu'une pipette graduée, une seringue, une spatule, un pinceau ou des compresses par exemple.

Les Inventeurs ont ainsi découvert, de façon particulièrement surprenante, que le traitement par voie topique des lésions cutanées associées à des maladies auto-immunes et/ou inflammatoires chez un sujet humain, sans effets secondaires notables, pouvait avantageusement utiliser
i. au moins le composé As₂O₅, éventuellement associé à d'autres composés de l'arsenic, comme décrit plus haut, ou, alternativement,
ii. un ou plusieurs desdits autres composés de l'arsenic.

L'utilisation de tels composés par voie topique apparaissait pourtant contre-intuitive dans la mesure où ces composés sont connus pour leur grande toxicité, et en particulier leur caractère irritant à l'origine de dermites de contact et d'autre irritation des muqueuses.

Ainsi, selon un autre aspect, il est décrit un composé de l'arsenic choisi parmi As₂O₃, As₂O₅, As₄O₆, As₂S₂, As₂S₃, As₂S₅ et As₄S₄, pour utilisation dans le traitement et/ou la prévention par voie topique des lésions cutanées associées à des maladies auto-immunes et/ou inflammatoires chez un sujet humain.

En d'autres termes, il est décrit l'utilisation d'un composé de l'arsenic choisi parmi As₂O₃, As₂O₅, As₄O₆, As₂S₂, As₂S₃, As₂S₅ et As₄S₄, pour la préparation d'un médicament pour le traitement et/ou la prévention par voie topique des lésions cutanées associées à des maladies auto-immunes et/ou inflammatoires chez un sujet humain, ainsi qu'une méthode de prévention et/ou de traitement par voie topique des lésions cutanées associées à des maladies auto-immunes et/ou inflammatoire comprenant l'administration d'un composé de l'arsenic choisi parmi As₂O₃, As₂O₅, As₄0₆, As₄O₆, As₂S₂, As₂S₃, As₂S₅ et As₄S₄ à un sujet humain.

Il est entendu au sens de l'invention que l'on ciblera les même pathologies que celles citées plus haut, à savoir les lésions cutanées associées aux maladies auto-immunes ou inflammatoires choisies parmi le lupus érythémateux, la sclérodermie, le psoriasis, la vascularite cutanée, le purpura vasculaire, les dermatoses bulleuses auto-immunes (en particulier la pemphigoïde bulleuse, la pemphigoïde cicatricielle, la dermatose à IgA linéaire, la dermatite herpétiforme, l'épidermo lyse bulleuse acquise, le pemphigus et ses variants), les dermatites, (en particulier la dermatite atopique, la dermatite séborrhéique, la dermatite de stase), la dermatomyosite, l'érythème noueux, le *pyoderma gangrenosum,* l'eczéma (en particulier l'eczéma atopique, l'eczéma de contact, l'eczéma dishydrosique), le lichen plan, le lichen scléro-atrophique, et l'alopécie areata. Préférentiellement les lésions cutanées associées à des maladies autoimmunes ou inflammatoires sont des lésions cutanées associées au lupus.

L'administration topique des composés de l'arsenic est facilitée par l'utilisation de compositions spécialement adaptées à cet usage. En effet, il est bien connu que la formulation d'une composition est déterminante pour garantir la bonne administration et l'efficacité d'un produit actif. Ceci est particulièrement vrai dans le cas de l'arsenic qui existe sous une grande variété de composés aux propriétés physico-chimiques différentes.

Selon un autre aspect, il est décrit une composition topique, en particulier dermatologique, comprenant au moins un composé de l'arsenic choisi parmi As₂O₃, As₂O₅, As₄O₆, As₂S₂, As₂S₃, As₂S₅ et As₄S₄, en particulier pour utilisation dans le traitement et/ou la prévention des lésions cutanées associées à des maladies auto-immunes chez un sujet humain.

Il est décrit des nécessaires ou kits comprenant au moins un contenant renfermant au moins un composé de l'arsenic choisi parmi As₂O₃, As₂O₅, As₄O₆, As₂S₂, As₂S₃, As₂S₅ et As₄S₄ sous une forme pharmaceutiquement acceptable pour son administration topique et/ou dermatologique. Le kit peut également comprendre les moyens d'application appropriés tels qu'une pipette graduée, et/ou une spatule.

Les souris MRL/Ipr développent spontanément un lupus érythémateux disséminé, une polyarthrite rhumatoïde et un syndrome de Sjögren caractérisés par des atteintes dermatologiques, vasculaires, rénales, pulmonaires, articulaires et oculaires dues à des infiltrats de cellules lymphoïdes. Comme dans l'espèce humaine, on observe chez les souris MRL/Ipr un fort dimorphisme sexuel, les souris femelles présentent une pathologie plus précoce et plus sévère que les mâles et meurent plus précocement. La maladie débute vers l'âge de 8 semaines et entraine la mort des souris, en moyenne, vers 18 semaines d'âge pour les femelles et 22 semaines pour les mâles, la durée de vie moyenne d'une souris normale étant de 2 ans.

### Expérience 1: Mise en évidence de l'efficacité thérapeutique du composé d'arsenic As₂O pour le traitement local des atteintes cutanées de la souris MRL/lpr ainsi que de sa bonne tolérance aux doses thérapeutiques envisagées.

But : Cette expérience vise à évaluer l'efficacité thérapeutique et la tolérance d'un traitement topique des atteintes cutanées sévères des souris auto-immunes MRL/lpr par le trioxyde d'arsenic ou As₂O₃. Elle a également pour objectif d'évaluer la possibilité d'adapter la posologie en fonction de la sévérité des atteintes tissulaires. Enfin, elle vise aussi à évaluer la rapidité du traitement en utilisant des souris considérés comme « âgées » au regard de la durée de vie limitée des souris MRL/lpr et donc susceptible de mourir avant d'avoir pu observer l'efficacité du traitement.

Méthodes : La solubilité des composés d'arsenic dépend principalement des conditions de pH, d'oxydoréduction et de température de l'excipient. Le composé d'arsenic As₂O₃ étant peu soluble dans l'eau, celui-ci est d'abord dissout à une forte concentration (60 à 70 mg/ml) dans une solution alcaline diluée (0,5 ou 1 M), et en chauffant légèrement, jusqu'à obtenir une solution limpide et incolore. Cette solution est rendue exempte de particules et stérile par une filtration sur un filtre de 0,22 µM. La solution mère d'As₂O₃ ainsi obtenue est ensuite diluée dans un tampon phosphate ou du sérum physiologique stérile à la concentration variant de 0,5 mg/ml en vue de son utilisation in vivo. La solution mère du composé d'arsenic As₂O₃ est conservée de 1 à 3 mois au réfrigérateur (4°C) alors que la solution diluée n'est conservée que 5 jours à 4°C et à l'abri de la lumière.

Des souris MRL/lpr (10 femelles et 10 mâles par groupe âgés de 4 à 5 mois) présentant des lésions cutanées sévères de type lupique, et pour certaines une vascularite cutanée nécrosante, ont été traitées, 3 ou 5 fois par semaine selon l'expérience (du lundi au vendredi), pendant 8 semaines, par l'application au niveau des lésions tissulaires d'une compresse (ou d'un pinceau) imprégnez d'une solution d'arsenic AS₂O₃ à des concentrations de 0,5 mg/ml. Les animaux témoins étaient des souris, de même âge et sexe, traitées par l'excipient.

Résultats : Les photos des Figures 1 et 2 sont caractéristiques du type et de l'étendue des lésions cutanées observées chez une souris MRL/lpr femelle (Figure 1) et une souris MRL/lpr mâle (Figure 2) au début du traitement (t0) et après 2, 4 et 8 semaines de traitement topique par le composé d'arsenic AS₂O₃ à raison de 5 applications/semaine.

Après 2 semaines de traitement, les destructions tissulaires ont cessées voire régressées, en particulier, pour ce qui concerne la nécrose des oreilles caractéristique d'une vascularite cutanée des vaisseaux (Figure 1). Après 8 semaines de traitement, on observe une disparition quasi complète des atteintes cutanées inflammatoires qui aboutit même à la repousse des poils. Aucun effet indésirable (immunosuppression non spécifique, toxicité tissulaire ou mort prématurée) n'a été observé chez les animaux traités par le composé d'arsenic As₂O₃ indiquant une bonne tolérance aux doses thérapeutiques utilisées. De plus, la grande majorité des animaux ont vécu près de 8 semaines après le début du traitement ce qui est considérable au regard de la durée de vie moyenne des souris MRL/lpr. A l'inverse, un grand nombre d'animaux témoins sont mort en cours d'expérience et tous les animaux survivants présentaient des lésions cutanées sévères après 8 semaines de traitement par l'excipient.

La maladie pouvant se manifester sous des formes plus ou moins sévères, il est nécessaire d'adapter la posologie. Dans l'expérience illustrée par la Figure 3, le nombre hebdomadaire d'applications topique du composé d'arsenic As₂O₃ a été ramené à 3 (lundi, mercredi, vendredi) chez des souris MRL/*lpr* dont la sévérité des atteintes cutanées était moindre que celles des animaux présentés aux Figures 1 et 2. L'évolution des lésions cutanées au cours de ce traitement a comme précédemment été suivie pendant 8 semaines. Comme le montre les photos de la Figure 3, une réduction significative des lésions tissulaires a été observée chez les souris MRL/*lpr* femelles après 8 semaines de traitement par 3 applications hebdomadaires du composé d'arsenic As₂O₃ au lieu de 5 pour les Figures 1 et 2. Il est donc tout à fait possible de moduler la posologie en fonction de la gravité des lésions observées en début de traitement.

Conclusions : Cette expérience montre que le composé d'arsenic As₂O₃ présente une grande efficacité thérapeutique par la voie topique dans le traitement des lésions inflammatoires sévères du tissu cutané, de la vascularite cutanée nécrosante, et de l'alopécie. De plus, il permet une régénération complète du tissu cutané objectivée par une repousse quasi complète des poils. Le composé d'arsenic As₂O₃ utilisé par la voie topique montre également une grande rapidité d'action puisque qu'après 2 semaines de traitement, les destructions du tissu cutané avaient cessées voire régressées. Pour les formes moins sévères de la maladie le nombre d'applications hebdomadaires peut être diminué sans perte significative d'efficacité. D'autre part, la majorité des animaux traités par le composé d'arsenic As₂O₃ ont vécu plus de 8 semaines après le début du traitement sans présenter d'effets indésirables notables indiquant que la tolérance du composé d'arsenic As₂O₃ est bonne aux doses thérapeutiques utilisées et que ce traitement topique à une influence favorable importante sur la longévité des souris MRL/lpr. En effet, à la fin de cette expérience, les animaux traités par le composé d'arsenic As₂O₃ avaient entre 24 et 28 semaines d'âge alors qu'ils meurent généralement entre 18 et 22 semaines d'âge.

### Expérience 2 : Marge thérapeutique et réduction progressive du nombre hebdomadaire d'applications topiques du composé d'arsenic As₂O₃

But : Cette expérience vise à évaluer la possibilité d'une décroissance progressive du nombre d'applications par semaine du composé d'arsenic d'As₂O₃ sans altérer l'efficacité du traitement topique. Cet espacement des doses s'avère nécessaire lorsque de fortes doses ont été utilisées lors des traitements d'attaque ou lorsque des effets indésirables surviennent chez un patient lors d'un traitement au long cours.

Méthodes : Les lésions cutanées sévères ainsi que les vascularites cutanées nécrosantes présentent avant traitement (temps t0 des Figures 3, 4 et 5) chez 15 souris MRL/lpr âgées de 4 mois ont été traitées par l'application d'une compresse (ou d'un pinceau) imprégnez d'une solution du composé d'arsenic As₂O₃ à la concentration de 0,5 mg/ml. Le nombre d'application par semaine a varié de 2 à 5 selon l'expérience. De même, la durée totale du traitement topique qui a variée de 7 à 32 semaines.

Résultats : 5 applications hebdomadaires (du lundi au vendredi) du composé d'arsenic As₂O₃ à la concentration de 0,5 mg/ml entraînent un arrêt de la progression des lésions tissulaires en 2 à 4 semaines selon le degré de gravité des lésions présentes au temps t0. Nous avons donc évalué les conséquences d'une réduction du nombre hebdomadaire d'applications du composé d'arsenic As₂O₃. Dans l'exemple présenté sur la Figure 4, le nombre d'applications topiques a été ramené à 2 par semaine (lundi et jeudi) et cela durant 5 semaines. La Figure 4 montre que non seulement cette réduction n'a pas entraînée une reprise des destructions tissulaires mais que les affections cutanées ont considérablement diminuées après 7 semaines de traitement topique.

Les résultats présentés sur la Figure 5 sont un autre exemple montrant qu'il est possible de moduler, au cours du traitement, le nombre hebdomadaires d'applications topiques du composé d'arsenic As₂O₃. Ainsi, une souris MRL/lpr femelle a d'abord été traitée durant 4 semaines à raison de 5 applications hebdomadaires (lundi au vendredi). Après l'arrêt de la progression des lésions tissulaires, le nombre d'application hebdomadaires a été ramené à 3 (lundi, mercredi, vendredi). Aucune rechute ou rebond de la maladie n'étant observé après 6 nouvelles semaines de traitement avec cette posologie réduite, le nombre d'application hebdomadaire a encore été réduit et ramené à 2 (lundi et jeudi) et cela durant 22 semaines supplémentaires. Au cours des 32 semaines qu'ont duré le traitement, des périodes sans traitement d'une dizaine de 10 jours ont été introduites. Il est important de rappeler que les souris MRL/lpr meurent entre l'âge de 4 et 5 mois. Or la souris MRL/lpr présentée Figure 5, et qui était âgée de 3 mois et demi au début du traitement (t0), a vécu jusqu'à l'âge de 1 an, date à laquelle nous l'avons euthanasiée.

Conclusion : Une fois qu'un arrêt de la progression des lésions tissulaires est obtenu avec un traitement d'attaque à base de 5 applications par semaine du composé d'arsenic As₂O₃, un espacement des doses peut alors être envisagé en réduisant le nombre hebdomadaire d'applications à 3 voire à 2 et cela pendant plusieurs semaines sans altérer l'efficacité du traitement par la voie topique. L'absence de rechute ou rebond de la maladie après diminution de la posologie démontre la grande efficacité de l'As₂O₃ topique ainsi que sa marge thérapeutique étendue. D'autre part cette expérience démontre comme l'expérience précédente qu'un traitement topique par le composé d'arsenic As₂O₃ à une influence positive sur la longévité des souris MRL/lpr.

### Expérience 3 : Mise en évidence de l'efficacité thérapeutique du composé d'arsenic As₂O₅ pour le traitement local des atteintes cutanées de la souris MRL/lpr ainsi que de sa bonne tolérance aux doses envisagées

But: La grande diversité étiologique des maladies auto-immunes et/ou inflammatoires rend nécessaire le développement d'un arsenal thérapeutique plus varié, en particulier, grâce à des molécules agissant par des mécanismes différents. Dans le cas de médicaments à base de composé d'arsenic, nous avons évalué l'efficacité thérapeutique du pentoxyde d'arsenic (As₂O₅) car sa solubilité dans l'eau, et donc son mode de préparation, ainsi que ses mécanismes d'action sont différents de ceux du trioxyde d'arsenic, As₂O₃. De plus, la toxicité du composé d'arsenic As₂O₅ est moindre que celle du composé AS₂O₃ (environ 60 fois plus faible) permettant ainsi de réduire les effets indésirables tout en conservant le bénéfice thérapeutique voire même d'augmenter la dose administrée, si nécessaire. L'expérience 3 vise donc à évaluer l'efficacité thérapeutique du composé d'arsenic As₂O₅ pour le traitement topique des atteintes cutanées sévères des souris auto-immunes MRL/lpr ainsi que son absence de toxicité. Elle vise également à évaluer la rapidité du traitement en utilisant des souris atteintes de lésions tissulaires sévères en début de traitement et donc susceptible de mourir rapidement au cours de l'expérience.

Méthodes : Le composé de l'arsenic As₂O₅ est d'abord dissout à une forte concentration (10 à 15 mg/ml) dans de l'eau distillée jusqu'à obtenir une solution limpide et incolore. La solution est rendue stérile et exempte de particules par une filtration sur un filtre de 0,22 µM. La solution mère d'As₂O₅ ainsi obtenue est ensuite diluée dans un tampon phosphate ou du sérum physiologique stérile pour obtenir une concentration comprise entre 0,5 mg/ml en vue de son utilisation in vivo. La solution mère du composé d'arsenic As₂O₅ est conservée de 1 à 3 mois au réfrigérateur (4°C) alors que la solution diluée n'est conservée que 5 jours à 4°C et à l'abri de la lumière.

Des souris MRL/lpr (12 femelles et 12 mâles âgés de 4 à 5 mois) présentant des lésions cutanées sévères de type lupique, et également de vascularite cutanée nécrosante pour certaines (temps t0 de la Figures 6), ont été traitées 5 fois par semaine (du lundi au vendredi), pendant 8 semaines, par l'application au niveau des lésions tissulaires d'une compresse (ou d'un pinceau) imprégnez d'une solution d'arsenic As₂O₅ à des concentrations de 0,5 mg/ml. Les animaux témoins étaient des souris, de même âge et sexe, traitées par l'excipient.

Résultats : La Figures 6 est un exemple illustrant la très grande efficacité thérapeutique du composé d'arsenic As₂O₅ ainsi que sa rapidité d'action. En effet, les photos présentées sur la Figure 6 montrent que les destructions cutanées avaient cessées dès la première semaine de traitement (à raison de 5 applications par semaine) et qu'elles avaient quasiment disparues après 4 semaines de traitement. A l'inverse, les animaux témoins traités par l'excipient présentaient toujours des lésions cutanées sévères.

Conclusions : Cette expérience montre que le composé d'arsenic As₂O₅ présente une grande efficacité thérapeutique par la voie topique dans le traitement des lésions inflammatoires sévères du tissu cutané, de la vascularite cutanée nécrosante, et de l'alopécie. Il induit aussi une régénération du tissu cutané lésée comme le montre la repousse quasi complète des poils. Il montre également une grande rapidité d'action puisque qu'en moins de 2 semaines de traitement, les destructions du tissu cutané avaient cessées voire régressées. Enfin, aucun effet indésirable, de type immunosuppression non spécifique, toxicité tissulaire ou mort prématurée, n'a été observé chez les animaux traités par l'As₂O₅ indiquant sa bonne tolérance aux doses thérapeutiques utilisées.

### Expérience 4 : Marge thérapeutique et réduction progressive du nombre hebdomadaire d'applications topiques du composé d'arsenic As₂O₅

But : Cette expérience vise à évaluer la possibilité d'une décroissance progressive du nombre hebdomadaire d'applications du composé d'arsenic As₂O₅ sans altérer l'efficacité du traitement topique. Cet espacement des doses s'avère nécessaire suite à un traitement d'attaque à fortes doses ou en cas de survenue d'effets indésirables liés au traitement au long court.

Méthodes : Les lésions cutanées sévères ainsi que les vascularites cutanées nécrosantes présentent au temps t0 chez 10 souris MRL/lpr âgées de 4 à 5 mois ont été traitées par l'application d'une compresse (ou d'un pinceau) imprégnez d'une solution du composé d'arsenic As₂O₅ à la concentration de 0,5 mg/ml. Le nombre hebdomadaire d'applications a progressivement été réduit de 5 à 1 pendant les 10 semaines qu'ont durée le traitement.

Résultats : Les animaux ont d'abord été traités par 5 applications hebdomadaires d'As₂O₅ (du lundi au vendredi). Comme illustré par la Figure 7, les lésions inflammatoires propres au tissu cutané ainsi que celles de vascularite ayant cessées après 2 semaines de traitement, nous avons ramené à 2 (lundi et jeudi) le nombre d'applications par semaine. Etant donné que les lésions cutanées avaient quasi disparue après 2 nouvelles semaines de traitement avec cette posologie réduite nous avons décidé de la réduire encore et de ramener à 1 le nombre hebdomadaire d'applications (le lundi) et ce jusqu'à l'euthanasie des animaux. Durant les 5 semaines de traitement par une seule application hebdomadaire aucune rechute ou rebond de la maladie n'ont été observés.

Conclusion : Cette expérience confirme la grande efficacité thérapeutique et rapidité d'action du composé d'arsenic As₂O₅ sur les lésions inflammatoires sévères du tissu cutané. En effet, l'arrêt de la progression des lésions tissulaires est généralement obtenu en moins de 2 semaines avec le traitement d'attaque à base de 5 applications par semaine. En conséquence, un espacement des doses peut être rapidement mis en place. Il a ainsi été possible de réduire de 5 à 1 le nombre hebdomadaire d'applications sans altérer l'efficacité du traitement par la voie topique. L'absence de rechute ou rebond de la maladie après cette diminution importante de la posologie démontre la grande efficacité de l'As₂O₅ topique ainsi que sa marge thérapeutique étendue. D'autre part, malgré la réduction importante de la posologie, la majorité des animaux traités par le composé d'arsenic As₂O₅ ont vécu plus de 8 semaines après le début du traitement indiquant que ce traitement topique à une influence favorable évidente sur la longévité des souris MRL/lpr.

### Expérience 5 : Mise en évidence de l'efficacité thérapeutique du composé d'arsenic As₂O₅ pour le traitement par la voie systémique des atteintes cutanées de la souris MRL/lpr ainsi que de sa bonne tolérance aux doses envisagées.

But: L'expérience 5 vise à évaluer l'efficacité thérapeutique du composé d'arsenic As₂O₅ administré par la voie systémique pour le traitement des atteintes cutanées sévères des souris auto-immunes MRL/lpr ainsi que son absence de toxicité.

Méthodes : Des souris MRL/lpr (6 femelles et 6 mâles âgés de 4 à 5 mois) présentant des lésions cutanées sévères de type lupique, et également de vascularite cutanée nécrosante (temps t0 des Figures 8A et 8B), ont été traitées 5 fois par semaine (du lundi au vendredi), pendant 3 ou 4 semaines, par des injections intrapéritonéales de 0,1 ml par 10 g de souris d'une solution d'arsenic As₂O₅ à la concentration de 0,5 mg/ml. Les animaux témoins étaient des souris, de même âge et sexe, traitées par l'excipient.

Résultats : La Figures 8 illustre, chez deux souris MRL/lpr mâles (partie A de la figure) et deux souris MRL/lpr femelles (partie B), la grande efficacité thérapeutique du composé d'arsenic As₂O₅ ainsi que sa rapidité d'action. En effet, les photos présentées sur la Figure 8 montrent que les destructions cutanées avaient, selon la sévérité des lésions initiales, fortement régressées voire quasiment disparues après 3 ou 4 semaines de traitement. A l'inverse, les animaux témoins traités par l'excipient présentaient toujours des lésions cutanées sévères.

Conclusions : Cette expérience montre que le composé d'arsenic As₂O₅ présente une grande efficacité thérapeutique par la voie systémique dans le traitement des lésions inflammatoires sévères du tissu cutané, de la vascularite cutanée nécrosante, et de l'alopécie. Il induit aussi une régénération du tissu cutané lésée comme le montre la repousse quasi complète des poils. Il montre également une grande rapidité d'action puisque qu'en 3 à 4 semaines de traitement, les destructions du tissu cutané avaient cessées voire régressées. Enfin, aucun effet indésirable, de type immunosuppression non spécifique, toxicité tissulaire ou mort prématurée, n'a été observé chez les animaux traités par l'As₂O₅ indiquant sa bonne tolérance aux doses thérapeutiques utilisées.

### Expérience 6 : Mise en évidence d'un effet synergique potentialisateur entre le composé d'arsenic As₂O₅ et le composé d'arsenic As₂O₃ pour le traitement ou la prévention des syndromes auto-immuns et lymphoprolifératif de la souris MRL/lpr.

But : Cette expérience vise à évaluer l'efficacité thérapeutique d'une association entre le composé d'arsenic As₂O₃ et le composé As₂O₅ pour le traitement par la voie topique et/ou systémique des atteintes tissulaires auto-immunes et inflammatoires des souris MRL/lpr.

Méthodes : Des souris MRL/lpr (mâles et femelles), présentant des lésions cutanées sévères de type lupique ainsi qu'une vascularite cutanée nécrosante ont été traitées 5 fois par semaine, durant 8 semaines, par l'administration d'une solution aqueuse contenant soit le composé d'arsenic As₂O₃ soit le composé As₂O₅ soit un mélange des deux composés As₂O₃ + As₂O₅ soit un tampon phosphate (PBS) comme contrôle négatif. Des doses peu efficaces (suboptimales) d'As₂O₃ ou d'As₂O₅ ont été volontairement utilisées. Les composés d'arsenic ont été administrés sous forme aqueuse à l'aide de compresses ou d'un pinceau pour un traitement locale soit par injection intrapéritonéale pour un traitement systémique.

A la fin du traitement, le sang de chaque animal a été prélevé afin de quantifier dans le sérum les taux d'auto-anticorps (anti-ADN, Facteur rhumatoïde) et des cytokines impliqués dans la maladie. La peau du dos de chaque animal a été prélevée en vue d'analyse histologique ainsi que pour l'analyse de la nature de l'infiltrat inflammatoire après isolement des cellules par des méthodes enzymatiques, leurs numérations au microscope et leurs analyses en cytométrie en flux à l'aide d'anticorps fluorescents spécifiques des leukocytes.

Résultats : Les Figures 9 et 10 montrent que l'efficacité thérapeutique d'un traitement local par une solution contenant le composé As₂O₃ associé au composé As₂O₅ (As₂O₃ + As₂O₅) est bien supérieure à celle d'une solution contenant seulement As₂O₃ ou As₂O₅ et cela quelque soit la voie d'injection : locale (Figure 9A) et systémique (Figure 9B). Les 24 souris MRL/lpr utilisées dans cette expérience présentaient au début du traitement (t0) des atteintes cutanées sévères et des lésions vasculaires de type vascularite cutanée responsable de la nécrose des oreilles et de la queue. La peau du dos de chaque animal a été isolée après 8 semaines de traitement et les cellules de l'infiltrat inflammatoire ont été isolées et quantifiées.

Les graphes présentés sur les Figures 9A et 9B donnent le nombre de cellules présentes dans l'infiltrat lymphocytaires au niveau des lésions cutanées du dos des souris après un traitement par un tampon phosphate (PBS), le composé As₂O₃, le composé As₂O₅ ou l'association As₂O₃ + As₂O₅ (3 souris par groupe). Les souris MRL/lpr ont volontairement été traitées avec des doses peu efficaces (suboptimales) d'As₂O₃ ou d'As₂O₅ comme le montre le nombre encore élevé de cellules dans l'infiltrat lymphocytaire à la fin du traitement (Figure 9) et la présence de lésions cutanées sévères (Figure 10). En revanche, quand les deux composés d'arsenic sont associés ensemble, et aux mêmes doses que lorsqu'ils sont administrés séparément, on observe une élimination quasi complète de l'infiltrat inflammatoire (Figure 9), avec une meilleure efficacité pour le traitement topique, ainsi qu'une disparition quasi complète des lésions tissulaires inflammatoires, attestée par la repousse des poils (Figure 10) démontrant une synergie de ces deux composés d'arsenic pour l'élimination de l'infiltrat lymphocytaire.

## Revendications

1. Composition comprenant une combinaison de composés de l'arsenic, **caractérisée en ce qu'**elle comporte entre 0,01% et 0,15% du composé d'arsenic As₂0₅ par rapport au poids total de la composition, et entre 0,05% et 5% du composé d'arsenic As203 par rapport au poids total de la composition.

2. Composition selon la revendication 1 **caractérisée en ce que** ladite combinaison consiste en le composé de l'arsenic As₂0₅ et le composé de l'arsenic supplémentaire As203.

3. Composition selon la revendication 1 ou 2, pour utilisation dans le traitement et/ou la prévention des lésions cutanées associées à des maladies auto-immunes et/ou inflammatoires chez un sujet humain.

4. Composition comprenant le composé de l'arsenic As₂0₅ et le composé de l'arsenic As₂O₃ pour utilisation dans le traitement et/ou la prévention des lésions cutanées associées à des maladies auto-immunes chez un sujet humain.

5. Composition selon l'une des revendications 1 à 2 ou composition pour utilisation selon les revendications 3 à 4,**caractérisée en ce qu'**elle est formulée sous une forme appropriée pour une administration topique, en particulier dermatologique.

6. Composition selon l'une des revendications 1 à 2 ou composition pour utilisation selon les revendications 3 à 4,**caractérisée en ce qu'**elle est formulée sous une forme appropriée pour une administration systémique.

7. Produits **caractérisés en ce qu'**ils contiennent un composé de l'arsenic As₂0₅ et le composé de l'arsenic supplémentaire As₂O₃ comme produit de combinaison pour une utilisation simultanée, séparée ou étalée dans le temps dans le traitement et/ ou la prévention des lésions cutanées associées à des maladies auto-immunes et/ou inflammatoires chez un sujet humain.

8. Produits pour une utilisation selon la revendication 7, dans laquelle l'application d'As₂O₅ est alternée avec l'administration du composé de l'arsenic As₂O₃.

## Patentansprüche

1. Zusammensetzung umfassend eine Kombination von Arsenverbindungen, **dadurch gekennzeichnet, dass** sie zwischen 0,01% und 0,15% der Arsenverbindung As₂O₅ bezogen auf das Gesamtgewicht der Zusammensetzung und zwischen 0,05% und 5% der Arsenverbindung As₂O₃ bezogen auf das Gesamtgewicht der Zusammensetzung enthält.

2. Zusammensetzung gemäß Anspruch 1, **dadurch gekennzeichnet, dass** die Kombination aus der Arsenverbindung As₂O₅ und der zusätzlichen Arsenverbindung As₂O₃ besteht.

3. Zusammensetzung gemäß Anspruch 1 oder 2 zur Verwendung in der Behandlung und/oder Prävention von Hautläsionen, die mit Autoimmun- und/oder Entzündungskrankheiten assoziiert sind, bei einem Menschen.

4. Zusammensetzung umfassend die Arsenverbindung As₂O₅ und die Arsenverbindung As₂O₃ zur Verwendung in der Behandlung und/oder Prävention von Hautläsionen, die mit Autoimmunkrankheiten assoziiert sind, bei einem Menschen.

5. Zusammensetzung gemäß einem der Ansprüche 1 bis 2 oder Zusammensetzung zur Verwendung gemäß den Ansprüchen 3 bis 4, **dadurch gekennzeichnet, dass** sie in einer für eine topische, insbesondere dermatologische, Verabreichung geeigneten Form formuliert ist.

6. Zusammensetzung gemäß einem der Ansprüche 1 bis 2 oder Zusammensetzung zur Verwendung gemäß den Ansprüchen 3 bis 4, **dadurch gekennzeichnet, dass** sie in einer für eine systemische Verabreichung geeigneten Form formuliert ist.

7. Produkte, **dadurch gekennzeichnet, dass** sie eine Arsenverbindung As₂O₅ und die zusätzliche Arsenverbindung As₂O₃ enthalten, als Kombinationsprodukt zur gleichzeitigen, getrennten oder über die Zeit verteilten Verwendung in der Behandlung und/oder Prävention von Hautläsionen, die mit Autoimmun- und/oder Entzündungskrankheiten assoziiert sind, bei einem Menschen.

8. Produkte zur Verwendung gemäß Anspruch 7, wobei die Anwendung von As₂O₅ mit der Verabreichung der Arsenverbindung As₂O₃ alterniert.

## Claims

1. A composition comprising a combination of arsenic compounds, **characterized in that** said combination comprises between 0.01% and 0.15% of the arsenic compound As₂O₅ relative to the total weight of the composition, and between 0.05% and 5% of the arsenic compound As₂O₃ relative to the total weight of the composition.

2. The composition according to claim 1, **characterized in that** said combination consists of the arsenic compound As₂O₅ and the additional arsenic compound As₂O₃.

3. The composition according to claim 1 or 2, for use in the treatment and/or prevention of skin lesions associated with autoimmune and/or autoinflammatory diseases in a human subject.

4. A composition comprising the arsenic compound As₂O₅ and the arsenic compound As₂O₃, for use in the treatment and/or prevention of skin lesions associated with autoimmune diseases in a human subject.

5. The composition according to one of claims 1 to 2 or the composition for use according to claims 3 to 4, **characterized in that** it is formulated in a form suitable for topical administration, in particular dermatological administration.

6. The composition according to one of claims 1 to 2 or the composition for use according to claims 3 to 4, **characterized in that** it is formulated in a form suitable for systemic administration.

7. Products containing an arsenic compound As₂O₅ and an additional arsenic As₂O₃, as a combination product for simultaneous, separate or spread over time use in the treatment and/or prevention of skin lesions associated with autoimmune and/or inflammatory diseases in a human subject.

8. Products for use according to claim 7, wherein application of AS₂O₃ is alternated with administration of arsenic compound As₂O₅.
